# EUROPÄISCHE PATENTSCHRIFT

(11) **EP 0 588 799 B1**
(45) Veröffentlichungstag und Bekanntmachung des Hinweises auf die Patenterteilung: **18.09.1996**
(21) Anmeldenummer: 92906808.8
(22) Anmeldetag: 20.03.1992
(51) Int. Cl.: C07C 311/29, A61K 31/18

(54) **NEUE SULFONAMIDE, VERFAHREN ZU IHRER HERSTELLUNG UND ARZNEIMITTEL, DIE DIESE VERBINDUNGEN ENTHALTEN**
NEW SULPHONAMIDES, PROCESS FOR PREPARING THE SAME AND MEDICAMENTS CONTAINING THESE COMPOUNDS
NOUVEAUX SULFAMIDES, LEUR PROCEDE DE PREPARATION ET MEDICAMENTS CONTENANT CES COMPOSES

(30) Priorität: 25.03.1991 DE 4109735
(43) Veröffentlichungstag der Anmeldung: 30.03.1994
(73) Patentinhaber: BOEHRINGER MANNHEIM GMBH, 68298 Mannheim (DE)
(72) Erfinder: HECK, Reinhard, D-6718 Grünstadt 3 (DE); DRESEL, Hans, A., D-6905 Schriesheim (DE)
(74) Vertreter: Weber, Manfred, Dr.
(86) Internationale Anmeldenummer: EP9200618
(87) Internationale Veröffentlichungsnummer: WO9216503

(56) Entgegenhaltungen:
- EP-A- 0 031 954
- EP-A- 0 221 344
- EP-A- 0 384 279
- BE-A- 845 158
- FR-A- 2 456 731

## Beschreibung

Die vorliegende Erfindung betrifft neue Sulfonamide der allgemeinen Formel I 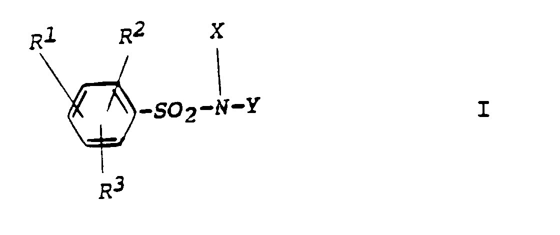 in der
- R¹: eine Hydroxyguppe,
- R² und R³,: die gleich oder verschieden sein können, einen geradkettigen oder verzweigten Alkylrest mit 1 bis 6 C-Atomen,
- X: ein Wasserstoffatom, oder einen C₁-C₆-Alkylrest,
und
- Y: einen geradkettigen oder verzweigten Alkylrest mit 1 bis 6 C-Atomen, einen Ar-(Cl-C6)-alkyl- oder Arylrest, wobei der Arylrest ein- bis dreifach in allen möglichen Positionen am Ring durch Hydroxy, Halogen, Trifluormethyl, C₁-C₆-Alkyl, Amino, C₁-C₆-Acylamino, Di(C₁-C₆)-Alkylamino, Nitro, oder Carboxyl substituiert sein kann, bedeuten,
sowie deren pharmakologisch unbedenkliche Salze, Verfahren zu ihrer Herstellung und Arzneimittel, die diese Verbindungen enthalten.
- R₁: steht bevorzugt in 2- oder 4-Stellung des Phenylrings.
- R₂ und R₃: bedeuten bevorzugt Wasserstoff, Methyl oder die tert-Butylgruppe.

Alkyl bedeutet in allen Fällen einen Rest mit 1 bis 6 C-Atomen, bevorzugt 1-4. Insbesondere sind hierunter Methyl, Ethyl, Propyl, Isopropyl, n-Butyl, tert. Butyl, Pentyl oder Hexyl zu verstehen.

Eine Aralkylgruppe bedeutet vorzugsweise Benzyl oder Phenethyl.

Eine Arylgruppe stellt vorzugsweise Phenyl dar.

Ein Acylrest bedeutet vorzugsweise Acetyl.

Für Y ist besonders bevorzugt der Isopropyl-, der n-Butyl-, der Benzyl- oder der Phenylrest, wobei dieser seinerseits in allen Positionen bevorzugt durch Hydroxy, Fluor, Chlor, Methyl, tert. Butyl, Amino, Acetylamino, Dimethylamino oder Nitro substituiert sein kann.

Einen dreifach substituierten Rest stellt insbesondere der 4-Hydroxy-3,5-di-tert.butyl-phenyl-Rest dar.

Die Verbindungen der vorliegenden Erfindung verfügen über eine starke Antioxidantienaktivität. Die Lipophilie dieser Antioxidantiengruppe bedingt, daß die Verbindungen sich im atherogenen low-density lipoprotein (LDL) anreichern und die sensitiven Bestandteile des LDL wirksam gegen reaktive Sauerstoffspezies schützen. Damit wird aber der LDL-Influx in die makrophagozytären Schaumzellen deutlich abgebremst; denn die pathologisch gesteigerte Aufnahme von atherogenem LDL in den Atheromzellen setzt dessen oxidative Modifikation voraus.

Antioxidantien sind Substanzen, die - allgemein gesehen - eine erhebliche Verzögerung der oxidativen Vorgänge bei einem zu schützenden Produkt bewirken. Ein potent antiatherosklerotisch wirksames Antioxidans ist Probucol®, das eine hypolipidämische Wirkung bei verschiedenen Tierspezies und am Menschen hat. Es ist ein sterisch gehindertes Alkylphenol, das im LDL akkumuliert. Im Tierversuch wurde gezeigt, daß Probucol die oxidative Modifikation von LDL in der Arterienwand blockiert und die Atherombildung direkt aufgrund der Antioxidansaktivität verhindert (D. Steinberg et al., Amer. J. Cardiol. 57, 16 M (1986).

Nachteile von Probucol® liegen in der geringen Resorption der Substanz, sowie in der extrem langen Verweildauer im Körpergewebe; die Ausscheidung von Probucol erfolgt hauptsächlich über die Faeces (vgl. M.N. Cayen, Pharmacol. Ther. 29, 157 (1985)).

Ferner senken Verbindungen der Formel I die Plasmalipide indem sie die intestinale Cholesterinresorption blockieren, in Konsequenz den intrahepatischen Pool des freien Cholesterins reduzieren und die Sekretion der nahrungsabhängigen Lipoproteine aus der Leber in das Plasma entsprechend vermindern. Die Inhibition der Cholesterinresporption beruht auf einer Hemmung der Acyl-Coenzym A: -cholesterintransferase (ACAT) Reaktion. In den Enterozyten katatysiert ACAT die Veresterung des Cholesterins, die notwendig ist, um Cholesterin in Chylomikronen einzpacken und über Darmlymphe und den Ductus thoracicus in das zirkulierende Blut einzuführen.

Die Substanzen verfügen über eine gute Resorption und inhibieren die ACAT abhängige Veresterung des freien Cholesterins nicht nur in den Enterozyten, sondern auch in den Zellen des Atheroms selbst. Sie verhindern dadurch deren schaumzellige Degeneration infolge Überladung mit Cholesterinester.

Die Verbindungen der Formel I finden aufgrund ihrer stabilisierenden Wirkung auf Lipoproteine Verwendung als Arzneimittel, insbesondere als Antiatherosclerotica.

Desweiteren wirken sie antibiotisch - besonders antibakteriell-, antiinflammatorisch, cytoprotektiv sowie antiasthmatisch. Sie können aber auch als Inhibitoren der reperfusionsabhängigen Lipidperoxidation und als Stabilisatoren des "lunq surfactant factor" eingesetzt werden.

Die Herstellung der Verbindungen der Formel I ist dadurch charakterisiert, daß man entweder ein Sulfonsäurechlorid der Formel II, in welcher R₁, R₂ und R₃ die oben angegebene Bedeutung haben, mit einem Amin der Formel III, in der X und Y die oben angegebene Bedeutung haben, zur Reaktion bringt oder ein Sulfonsäureamid der Formel IV, in der R₁, R₂, R₃ und X die angegebene Bedeutung haben, mit einer Verbindung der Formel V, in der Y die oben angegebene Bedeutung hat und A einen reaktiven Rest, wie z. B. Halogen, Sulfonsäureester etc. darstellt, umsetzt.

Die Reaktionen werden vorwiegend bei Raumtemperatur in einem chemisch inerten Lösungsmittel wie CH₂Cl₂, Toluol o.ä. vorzugsweise in Gegenwart eines säurebindenden Reagens, wie z.B. Pyridin, Triethylamin durchgeführt (z.B. analog F. Muth in Houben-Weyl, Bd. 9 S. 613). 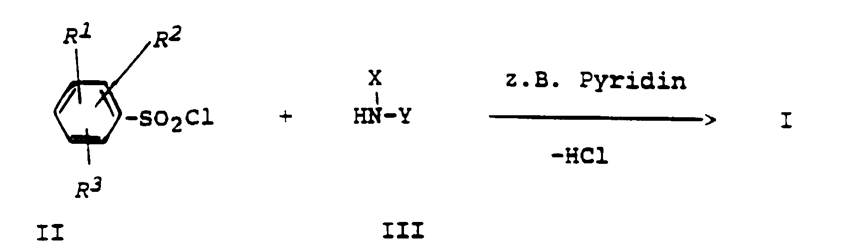 oder 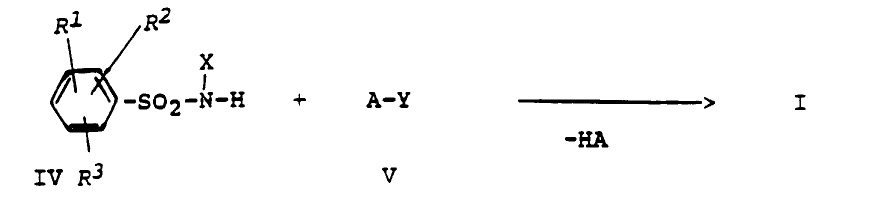 Falls die so gewonnenen Verbindungen der Formel I nicht schon das Endprodukt darstellen, kann am Sulfonamid-Stickstoff ggf. mit Alkylhalogeniden (z.B. Methyliodid) zusätzlich der Rest X (z.B. CH₃) eingeführt werden.

Die notwendigen Ausgangsmaterialien II und III bzw. (IV und V) sind meist literaturbekannt, bzw. nach üblichen Verfahren analog herstellbar (detaillierte Angaben vgl. Beispiele).

Falls einzelne Reaktionsprodukte nicht in genügender Reinheit anfallen, kann die Reinigung der Rohprodukte durch Kristallisation oder Säulenchromatographie erfolgen.

Zur Herstellung von Salzen mit physiologisch verträglichen organischen oder anorganischen Basen wie beispielsweise Natriumhydoxid, Kaliumhydroxid, Calciumhydroxid, Ammoniumhydroxid, Methylglucamin, Morpholin, Triethylamin oder Ethanolamin können die Verbindungen der Formel I mit den entsprechenden Basen umgesetzt werden. Auch Mischungen der sauren Verbindungen mit einem geeigneten Alkalicarbonat bzw.-hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder öl, wie z. B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z. B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z. B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 10 mg/kg Körpergewicht.

Bevorzugt im Sinne der Erfindung sind außer den in den Beispielen genannten Verbindungen der Formel I die folgenden:

### Beispiel 1

### a) 3,5-Di-^{t}butyl-4-hydroxy-benzolsulfochlorid (Rohgemisch)

Zu einer Lösung von 30.9 g (0.15 mmol) 2,6-Di-^{t}butylphenol in 100 ml Dichlormethan werden bei 0° C ein Gemisch von 38 ml (0.6 mol) Chlorsulfonsäure und 50 ml Dichlormethan innerhalb 1 h zugetropft.

Man rührt 1 h bei 0° C nach und gießt anschliepend die Mischung auf 200 ml Eiswasser. Nach Abtrennen der organischen Phase wird diese noch 3-fach mit Wasser gewaschen und über MgSO₄ getrocknet. Nach Abziehen des Lösungsmittels i. Vak. verbleibt ein öliges Sulfochlorid-Rohgemisch. 20.0 g, violett gefärbtes öl.

### b) (3,5-Di-^{t}butyl-4-hydroxy)benzolsulfo-benzylamid

6.3 ml (46 mmol) Benzylamin werden in 80 ml Toluol gelöst und dazu ein Gemisch von 7.0 g (23 mmol) des unter a) erhaltenen Sulfochlorids, gelöst in 10 ml Toluol, langsam zugetropft. Man rührt 2 h bei Raumtemperatur nach, versetzt anschließend mit 50 ml Wasser, trennt die Phasen und schüttelt die Toluolphase noch 2-fach mit je 50 ml Wasser aus. Die organische Phase wird über Na₂SO₄ getrocknet und i. Vak. eingedampft.

Das so erhaltene Rohöl wird an einer Kieselgel-Mitteldrucksäule mit Hexan/Essigester (9:1) als Laufmittel gereinigt. In der Reihenfolge der Elution erhält man, nach Abziehen des Lösungsmittels i. Vak.:

### 1. (3,5-Di-^{t}butyl-2-hydroxy)benzolsulfo-benzylamid, 0.6 g, als farblose Kristalle, Fp: 94° C, als Nebenprodukt, sowie,

### 2. (3,5-Di-^{t}butyl-4-hydroxy)benzolsulfo-benzylamid, 1.6 g, farblose Kristalle, Fp: 89° C

### Beispiel 2

### 1) 3-^{t}Butyl-2-hydrozy-5-methyl-benzolsulfochlorid

Eine Lösung von 10.8 g (60 mmol) 2,6-Di-tbutyl-4-methyl-phenol in 40 ml Dichlormethan wird bei 0° C unter Rühren innerhalb einer Stunde mit 8.8 ml (130 mmol) Chlorsulfonsäure versetzt. Anschließend wird 50 h bei Raumtemperatur gerührt und darauf in Eiswasser gegossen. Nach Abtrennen der organischen Phase wird diese noch 2-fach mit Wasser gewaschen und über MgSO₄ getrocknet. Nach Einengen der Lösung verbleibt ein kristallines Rohprodukt, das zur Weiterverarbeitung ohne weitere Reinigung geeignet ist. 7.5 g, schwach violette Kristalle vom Fp: 43-48° C
Lit.: modifiziert nach: A. H. Weinstein, J. Org. Chem. 1967, 3669.

### 2) (3-^{t}Butyl-2-hydroxy-5-methyl)benzolsulfo-benzylamid

3.3 ml (31 mmol) Benzylamin werden mit 30 ml Toluol versetzt und dazu ein Gemisch des unter a) beschriebenen Sulfochlorids in 20 ml Toluol getropft. Nach 14 h Rühren der Reaktionsmischung bei Raumtemperatur wird die organische Phase 2-fach mit 2 n HCL und darauf mit Wasser geschüttelt, die Toluolphase abgetrennt und über MgSO₄ getrocknet. Nach Abziehen des Lösungsmittels i. Vak. wird der kristalline Rückstand mit Ligroin angerieben und abgesaugt. 3.9 g, farblose Kristalle, Fp: 108° C.

### Beispiel 3

In analoger Weise zu den in den Beispielen 1 und 2 genannten Verbindungen erhält man (siehe Tabelle):

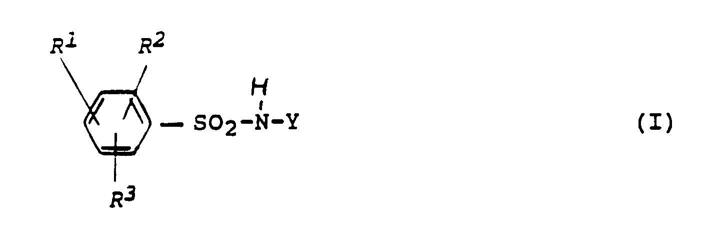

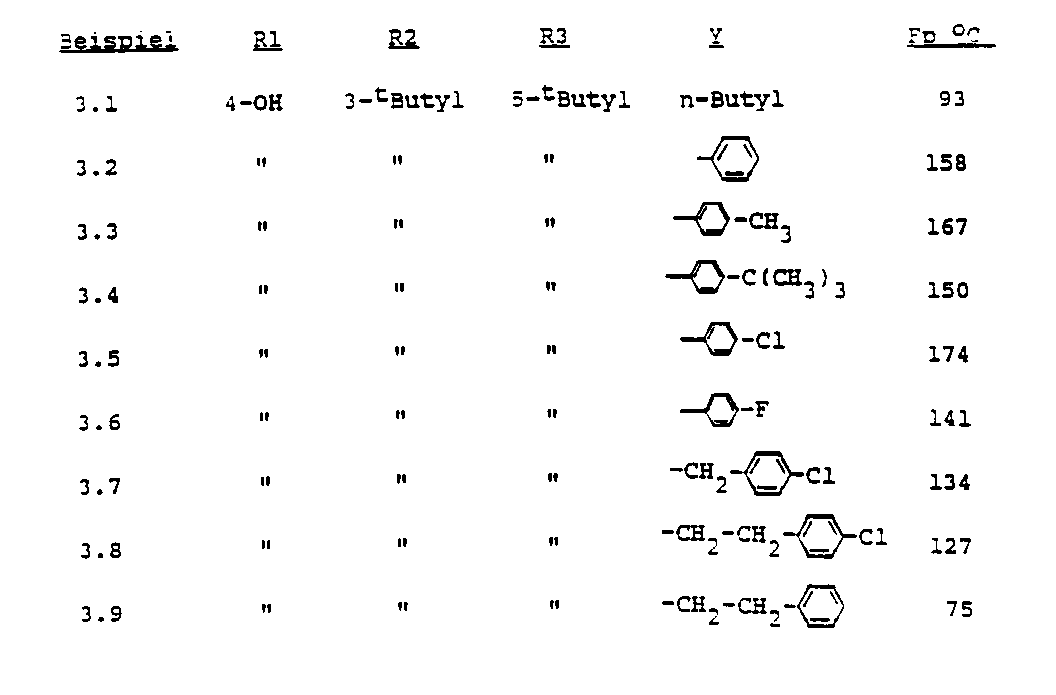

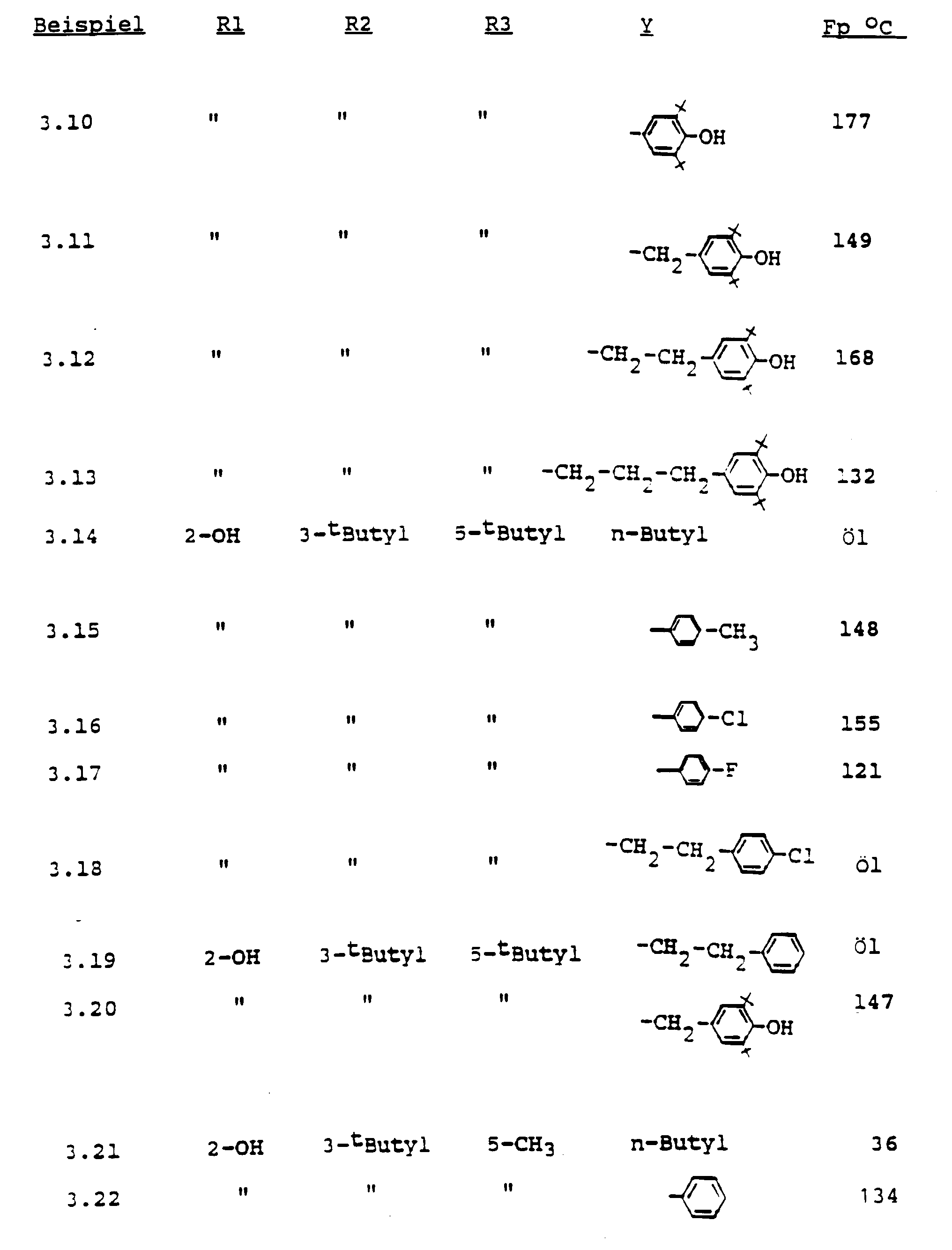

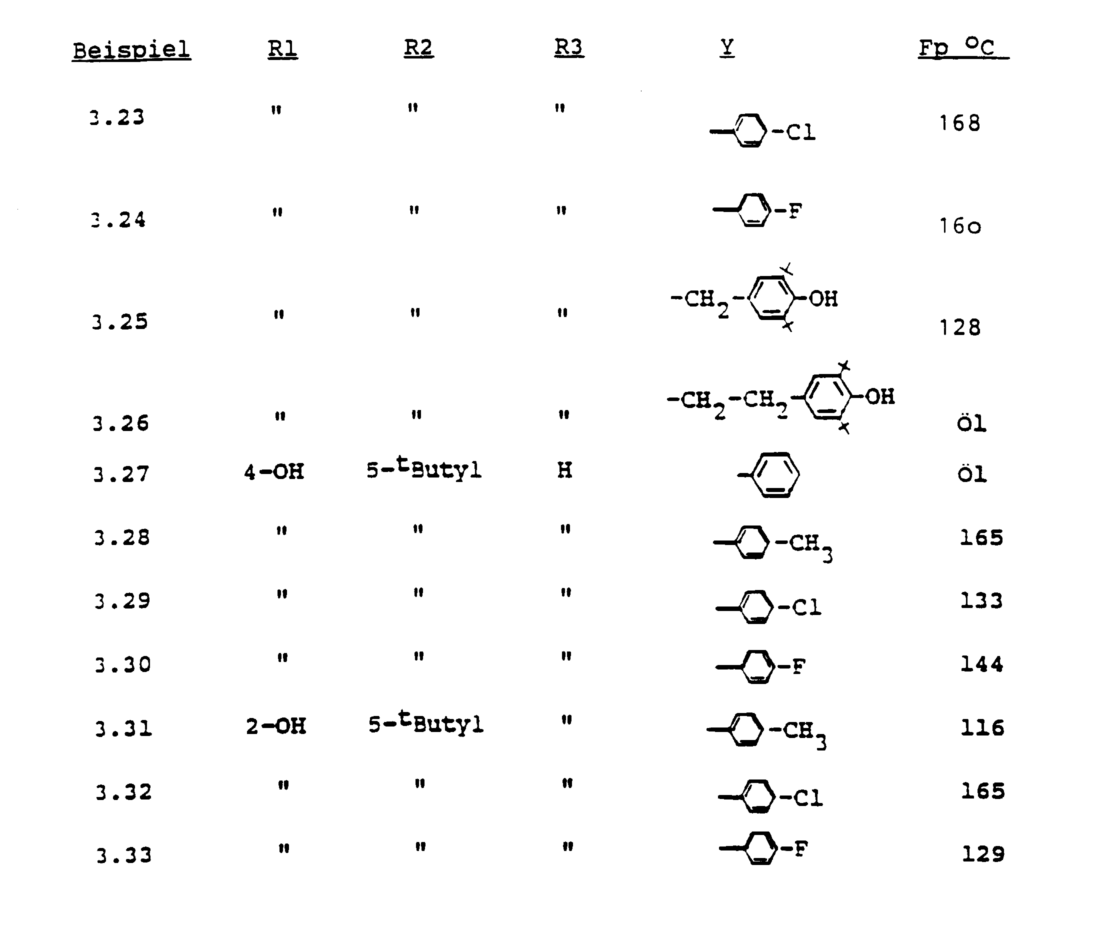

### Pharmakologischer Versuchsbericht

Eine antiatherosklerotische Aktivität der erfindungsgemäßen Verbindungen wird durch die nachstehend näher beschriebenen Tests belegt, deren Ergebnisse in der Tabelle zusammengefaßt sind.

**Tabelle**

| In vitro Wirkungen der Sulfonamidanaloga | | | |
|---|---|---|---|
| Verbindung Bsp. Nr. | Hemmung der Cholesterinesterbildung in Macro- (P388D.1) | Stabilisierung v. Plasma LDL: Verlängerung der "lag-Phase" | Hemmung der 15-Lipoxygenase vermittelten LDL-Oxidation |
| 3.03 | -71.2 | 10 | |
| 3.05 | -59.5 | 12 | |
| 3.09 | -73.9 | 12 | |
| 3.10 | -77.4 | | 0.3 |
| 3.11 | -66.7 | 79 | 1 |
| 3.12 | -63.4 | | 0.5 |
| 3.13 | -72.6 | | 1 |
| 3.20 | -55.9 | 92 | |
| 1b).2 | -73,0 | 8 | |
| 2.2 | -64.1 | | 6 |

Methoden zur oben stehenden Tabelle "In vitro Wirkung der sulfonamidanaloga"

### Hemmung der Cholesterinesterbildung in Makrophagen (nach Huber et al. (1990) Free Rad. Res. Comms. 8, 167-173)

Humanplasma-LDL wurde durch sequentielle Ultrazentrifugation bei präselekierten Dichten aus dem Plasma gesunder männlicher Nichtraucher isoliert und für 5 h bei 37° C in F-10 Medium + 1µM Cu(II) zur Depletion seiner lipophilen Antioxidantien inkubiert. Die Bildung von Cholesterinestern in P 388 D.1 Makrophagen unter Einfluß von konditioniertem human Plasma-LDL wurde nach 18 h Inkubation der Zellen (2 x 10⁶/ml) in F-10 Medium + 1 µM Cu(II) + 50 µg/ml LDL + 5 % FCS nach Extraktion der Neutral fette dünnschichtchromatographisch gemessen.

Referenzsubstanz: Probucol, 10 /uM Hemmung der Chol-ester-bildung 25-42 % (n=7).

### Stabilisierung von Plasma-LDL (nach Huber et al. (1990) Free Rad. Res. Comms. 8, 167-173)

LDL (125 µg Protein/ml) wurde in 20 mM Tris-Cl pH 8, 150 mM NaCl, 10 µM CuCl₂ mit und ohne Testverbindung (gelöst in Äthanol, Äthanolendkonzentration 0,5 %) bei 42° C oxidiert. Es wurden jeweils 3 Moleküle Testsubstanz pro LDL-Partikel eingesetzt. Die Oxidation wurde kontinuierlich photometrisch bei A 234 durch die Messung der Dienbildung verfolgt. Die sogenannte "lag Phase" ist als dasjenige Zeitintervall definiert, das sich aus dem Schnittpunkt der Tangente der Kurve während der Propagationsreaktion der Lipidperoxidation und der Zeitachse ergibt.

Referenzsubstanz: Probucol, 3 Moleküle/LDL-Partikel verlängern die lag-Phase von LDL um 10 %.

### Hemmung der Sojabohnenlipoxygenase (15-Lipoxygenase) am LDL-Substrat (nach Cathcart et al. (1991) J. Lipid Res. 32, 63-70)

LDL (100 µg Protein/ml) wurde in 20 mM Tris-Cl pH 8,3 150 mM NaCl mit und ohne Testsubstanz (gelöst in Äthanol, Endkonzentration des Äthanol 0,5 %) mit 5.000 U/2 ml Sojabohnen-Lipoxygenase (Sigma, München) bei 37° C inkubiert: Die Oxidation wurde durch Messung der Diene kontinuierlich bei A 234 verfolgt. Als IC 50-Wert wird diejenige Konzentration der Testverbindung angegeben, die zur Halbierung der im Testansatz über die Zeit linearen Dienbildung führt.

Referenzsubstanz: Probucol, CI 50 = 20 µM.

## Patentansprüche

1. Verbindungen der Formel I 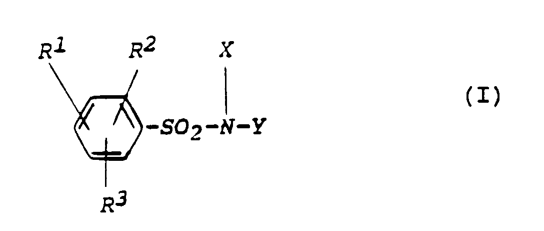 in der
R₁ eine Hydroxygruppe,
R₂ und R₃, die gleich oder verschieden sein können, einen geradkettigen oder verzweigten Alkylrest mit 1 bis 6 C-Atomen,
X ein Wasserstoffatom oder einen C₁-C₆-ASky-rest und
Y einen geradkettigen oder verzweigten Alkylrest mit 1 bis 6 C-Atomen, einen Ar-(C₁-C₆)-aBky- oder Arylrest, wobei der Arylrest ein- bis dreifach in allen möglichen Positionen am Ring durch Hydroxy, Halogen, Trifluormethyl, C₁-C₆-Alkyl, Amino, C₁-C₆-Acylamino, Di(C₁-C₆)Alkylamino, Nitro oder Carboxyl substituiert sein kann,
bedeuten, sowie deren pharmakologisch unbedenklichen Salze.

2. Verfahren zur Herstellung von Verbindungen der Formel I 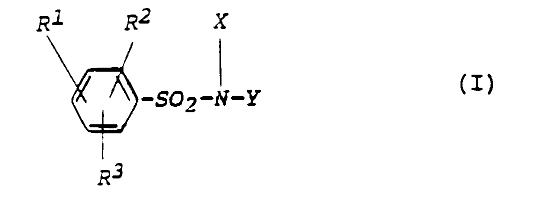 in der
R₁ eine Hydroxygruppe,
R₂ und R₃, die gleich oder verschieden sein können, einen geradkettigen oder verzweigten Alkylrest mit 1 bis 6 C-Atomen,
X ein Wasserstoffatom oder einen C₁-C₆-ASky-rest und
Y einen geradkettigen oder verzweigten Alkylrest mit 1 bis 6 C-Atomen, einen Ar-(C₁-C₆)-aBky- oder Arylrest, wobei der Arylrest ein- bis dreifach in allen möglichen Positionen am Ring durch Hydroxy, Halogen, Trifluormethyl, C₁-C₆-Alkyl, Amino, C₁-C₆-Acylamino, Di(C₁-C₆)Alkylamino, Nitro oder Carboxyl substituiert sein kann,
bedeuten,
sowie deren pharmakologisch unbedenklichen Salze,
dadurch gekennzeichnet, daß man in an sich bekannter Weise entweder
a) ein Sulfonsäurechlorid der Formel II 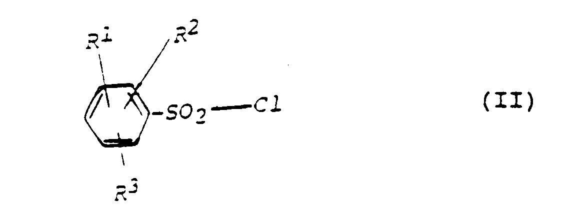 in der R₁, R₂ und R₃ die angegebene Bedeutung haben,
mit einem Amin der Formel III 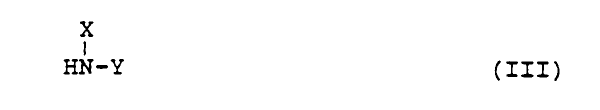 in der X und Y die angegebene Bedeutung haben umsetzt, oder
b) ein Sulfonamid der Formel IV 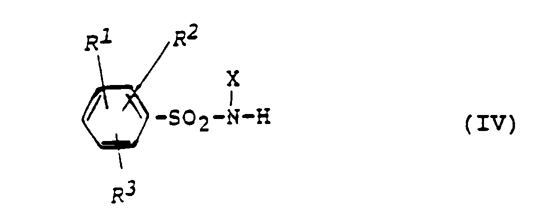 in der R₁, R₂, R₃ und X die angegebene Bedeutung haben,
mit einer Verbindung der Formel V
A-Y (V)
in der Y die angegebene Bedeutung hat und A einen reaktiven Rest darstellt,
umsetzt,
und anschließend gegebenenfalls die erhaltenen Verbindungen der Formel I, in der X=Wasserstoff bedeutet, nachträglich alkyliert, und gegebenenfalls die Verbindungen in pharmakologisch verträgliche Salze überführt.

3. Arzneimittel, enthaltend mindestens eine Verbindung gemäß Anspruch 1 neben üblichen Träger- und Hilfsstoffen.

4. Verwendung von Verbindungen gemäß Anspuch 1 zur Herstellung von Arzneimitteln als Antiatherosclerotica.

## Claims

1. Compounds of the formula I 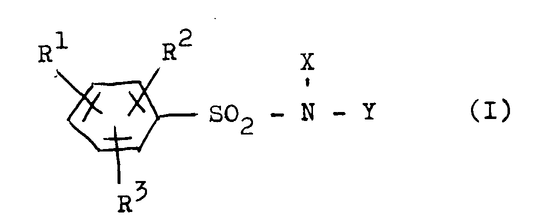 in which R₁ signifies a hydroxyl group, R₂ and R₃, which can be the same or different, a straight-chained or branched alkyl radical with 1 to 6 C-atoms, X a hydrogen atom or a C₁-C₆-alkyl radical and Y a straight-chained or branched alkyl radical with 1 to 6 C-atoms, an Ar-(C₁-C₆)-alkyl or aryl radical, whereby the aryl radical can be substituted one to three times in all possible positions on the ring by hydroxyl, halogen, trifluoromethyl, C₁-C₆-alkyl, amino, C₁-C₆-acylamino, di-(C₁-C₆)alkylamine, nitro or carboxyl, as well as their pharmacologically compatible salts.

2. Process for the preparation of compounds of the formula I 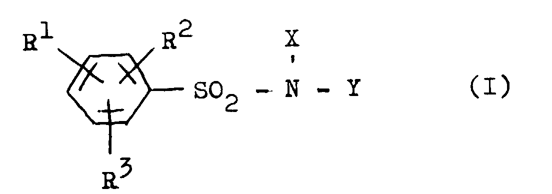 in which R₁ signifies a hydroxyl group, R₂ and R₃, which can be the same or different, a straight-chained or branched alkyl radical with 1 to 6 C-atoms, X a hydrogen atom or a C₁-C₆-alkyl radical and Y a straight-chained or branched alkyl radical with 1 to 6 C-atoms, an Ar-(C₁-C₆)alkyl or aryl radical, whereby the aryl radical can be substituted one to three times in all possible positions of the ring by hydroxyl, halogen, trifluoromethyl, C₁-C₆-alkyl, amino, C₁-C₆-acylamino, di-(C₁-C₆)-alkylamino, nitro or carboxyl, as well as of their pharmacologically compatible salts, characterised in that, in per se known manner, one either
a) reacts a sulphonic acid chloride of the formula II 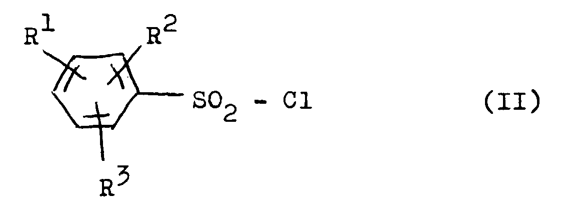 in which R₁, R₂ and R₃ have the given meaning, with an amine of the formula III 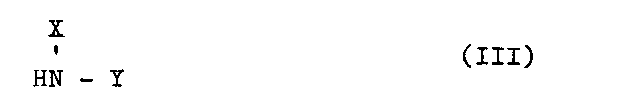 in which X and Y have the given meaning, or
b) reacts a sulphonamide of the formula IV 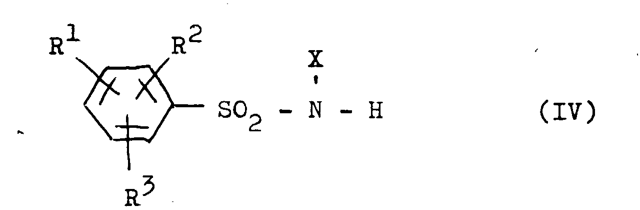 in which R₁, R₂, R₃ and X have the given meaning, with a compound of the formula V
A - Y (V)
in which Y has the given meaning and A represents a reactive residue,
and subsequently possibly alkylates the compounds obtained of the formula I, in which X = hydrogen, and possibly converts the compounds into pharmacologically compatible salts.

3. Medicaments containing at least one compound according to claim 1, besides usual carrier and adjuvant materials.

4. Use of compounds according to claim 1 for the preparation of medicaments as anti-atherosclerotics.

## Revendications

1. Composés de formule I 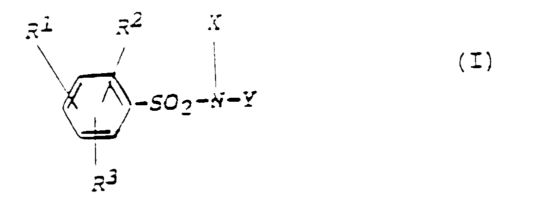 dans laquelle
R₁ représente un groupe hydroxyle,
R₂ et R₃ qui peuvent être identiques ou différents, représentent un reste alkyle à chaîne linéaire ou ramifiée, ayant de 1 à 6 atomes de C,
X représente un atome d'hydrogène ou un reste alkyle en C₁-C₆, et
Y représente un reste alkyle à chaîne linéaire ou ramifiée, ayant de 1 à 6 atomes de C, un reste Ar-(alkyle en C₁-C₆) ou aryle, le reste aryle pouvant être substitué de une à trois fois, dans toutes les positions possibles du cycle, par un groupe hydroxy, halogéno, trifluorométhyle, alkyle en C₁-C₆, amino, acylamino en C₁-C₆, di(alkyle en C₁-C₆)amino, nitro ou carboxyle,
ainsi que leurs sels pharmacologiquement acceptables.

2. Procédé de préparation de composés de formule I 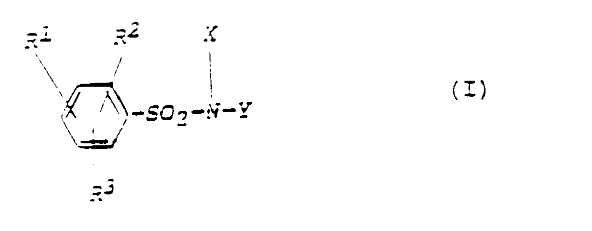 dans laquelle
R₁ représente un groupe hydroxyle,
R₂ et R₃ qui peuvent être identiques ou différents, représentent un reste alkyle à chaîne linéaire ou ramifiée, ayant de 1 à 6 atomes de C,
X représente un atome d'hydrogène ou un reste alkyle en C₁-C₆, et
Y représente un reste alkyle à chaîne linéaire ou ramifiée, ayant de 1 à 6 atomes de C, un reste Ar-(alkyle en C₁-C₆) ou aryle, le reste aryle pouvant être substitué de une à trois fois, dans toutes les positions possibles du cycle, par un groupe hydroxy, halogéno, trifluorométhyle, alkyle en C₁-C₆, amino, acylamino en C₁-C₆, di(alkyle en C₁-C₆)amino, nitro ou carboxyle,
ainsi que de leurs sels pharmacologiquement acceptables,
caractérisé par le fait que, de manière connue en soi, on fait réagir, soit
a) un chlorure d'acide sulfonique de formule II 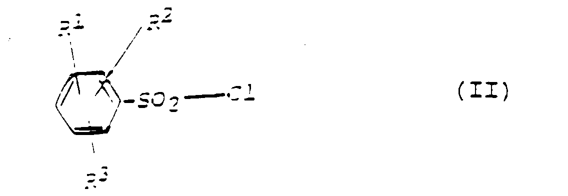 dans laquelle R₁, R₂ et R₃ ont les significations mentionnées, avec une amine de formule III 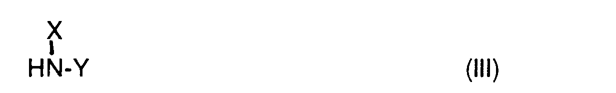 dans laquelle X et Y ont les significations mentionnées, soit
b) un sulfonamide de formule IV 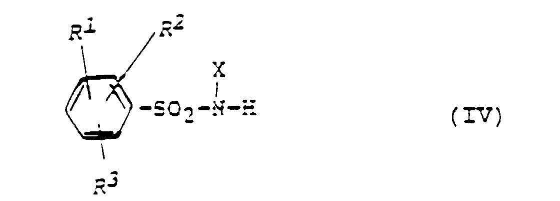 dans laquelle R₁, R₂, R₃ et X ont les significations mentionnées,
avec un composé de formule V
A-Y (V)
dans laquelle Y a la signification mentionnée et A représente un reste réactif,
et ensuite, on soumet éventuellement les composés de formule I obtenus, où X = un atome d'hydrogène, à une alkylation ultérieure, et on transforme éventuellement les composés en leurs sels pharmacologiquement acceptables.

3. Médicament, contenant au moins un composé selon la revendication 1, en plus de véhicules et adjuvants pharmacologiquement acceptables.

4. Utilisation de composés selon la revendication 1 pour la préparation de médicaments anti-athérosclérotiques.
